# EUROPÄISCHE PATENTSCHRIFT

(11) **EP 1 099 730 B1**
(45) Veröffentlichungstag und Bekanntmachung des Hinweises auf die Patenterteilung: **03.09.2003**
(21) Anmeldenummer: 00811039.7
(22) Anmeldetag: 06.11.2000
(51) Int. Cl.: C09D 5/03, C09D 167/00, C09D 133/06, C08G 65/32

(54) **Härtbare Zusammensetzung**
Curable composition
Composition durcissable

(30) Priorität: 10.11.1999 CH 205699
(43) Veröffentlichungstag der Anmeldung: 16.05.2001
(73) Patentinhaber: Vantico AG, 4057 Basel (CH)
(72) Erfinder: Rickert, Christoph, 4153 Reinach (CH); Turpin, François, 68330 Huningue (FR); François, Jacques, 68300 Saint Louis (FR); Tena, Mireille, 4310 Rheinfelden (CH)
(74) Vertreter: Dannappel, Hans-Jochen, Dr.

(56) Entgegenhaltungen:
- WO-A-94/14906
- US-A- 5 770 268
- DATABASE WPI Section Ch, Week 199416 Derwent Publications Ltd., London, GB; Class A81, AN 1994-133020 XP002155774 & SU 1 792 956 A (UKR PLASTICS RES INST), 7. Februar 1993 (1993-02-07)

## Beschreibung

Die Erfindung betrifft eine Pulverlackzusammensetzung enthaltend ein Bindemittel, das ausgewählt ist aus Carboxylgruppen enthaltenden Polyestem, Carboxylgruppen enthaltenden Poly(meth)acrylaten und Gemischen der genannten Substanzen, und eine oder mehrere Epoxidverbindungen als thermischen Härter, sowie ein bevorzugtes Herstellungsverfahren für einen Typ der zu Anwendung kommenden Epoxidverbindungen.

Pulverlackzusammensetzungen, wie eingangs genannt, werden in vielfältiger Form eingesetzt. Besonders für Aussenanstriche, die eine hohe Wetterfestigkeit aufweisen müssen, hat sich hierbei Triglycidylisocyanurat (TGIC) als Epoxidhärter durchgesetzt. Unter anderem seine feste Konsistenz hat dazu geführt, dass TGIC heute als Standardhärter für Pulverlacke auf Basis von Carboxylgruppen enthaltenden Polyestem als Bindemittel (s. z. B. Ullmann's Encyclopedia of Industrial Chemistry, 5th Ed., Vol A9, p. 559) und von Carboxylgruppen enthaltenden Poly(meth)acrylaten (s. z. B. Johnson Wax Speciality Chemicals Product Application Bulletin, Powder Coatings) gilt.

Seit einiger Zeit sind jedoch auch aussenbewitterungsstabile Pulverlacke auf Basis einer von TGIC freien, festen Mischung von Epoxidharzen als Härter bekannt (s. z. B. in der EP-A-0 536 085), wobei wesentliche Anteile eines flüssigen, höher funktionellen Epoxidharzes, z. B. von Trimellitsäuretriglycidylester, in ein festes Epoxidharz, z. B. Diglycidylterephthalat, eingearbeitet werden, ohne dass das Gesamtgemisch aus Epoxidharzen dadurch flüssige Konsistenz erhält. In der industriellen Praxis stehen bisher jedoch fast nur difunktionelle Glycidylester als Festharze für derartige Härtergemische zur Verfügung. Ausserdem macht das Festharz den grössten Teil dieser Gemisches aus, so dass es sich als ein wesentlicher Nachteil dieser Härtergemische erweist, dass ihre Epoxidfunktionalität im Vergleich zu TGIC stark reduziert ist. Weiterhin ist es nicht einfach, 1,2-Dicarbonsäuren im industriellen Massstab sauber zu glycidylisieren.

Somit besteht weiterhin ein Bedarf an neuartigen Pulverlacken mit vergleichbaren lacktechnischen Eigenschaften wie die oben genannten Pulverlackzusammensetzungen, d. h. insbesondere mit gutem Verlauf und hoher Reaktivität und der Möglichkeit zur Herstellung von Beschichtungen mit hoher Vemetzungsdichte und hoher Wetter- und UV Stabilität. Die vorliegende Erfindung stellt solche neuartigen Pulverlacke zur Verfügung.

Speziell betrifft die Erfindung Pulverlackzusammensetzungen, die ein Bindemittel, das ausgewählt ist aus Carboxylgruppen enthaltenden Polyestern, Carboxylgruppen enthaltenden Poly(meth)acrylaten und Gemischen der genannten Substanzen, sowie eine oder mehrere Epoxidverbindungen enthalten und die dadurch gekennzeichnet sind, dass die Epoxidverbindungen mindestens eine bei 25°C feste Verbindung der Formel (I) umfassen: 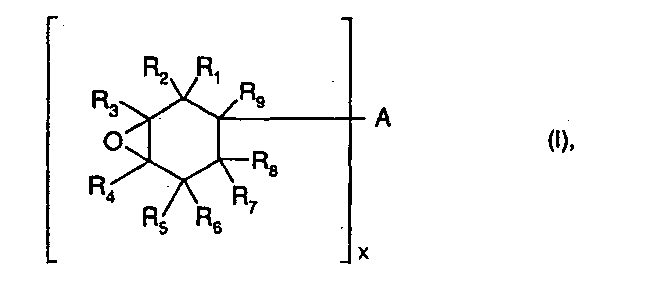 worin
- A: einer Gruppe der Formel (II), (III), (IV) oder (VI) entspricht: 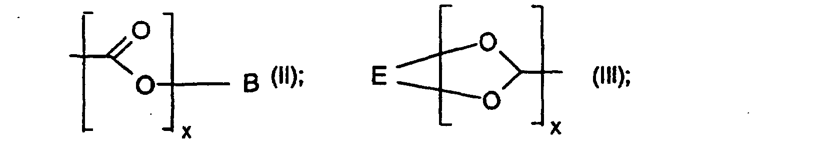 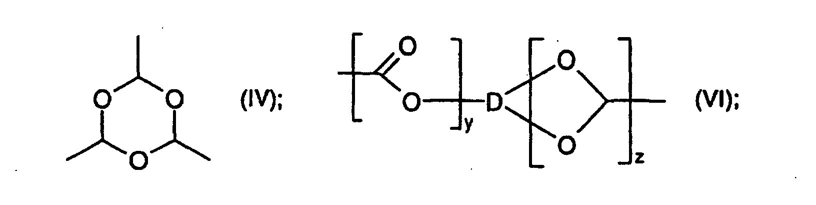
worin
- B: einen x-wertigen organischen Rest darstellt, abgeleitet von einem Polyol mit x oder mehr Hydroxylgruppen durch Entfernung von x Hydroxylgruppen;
- E: einen (2x)-wertigen organischen Rest darstellt, abgeleitet von einem Polyol mit (2x) oder mehr Hydroxylgruppen durch Entfernung von (2x) Hydroxylgruppen; und
- D: einen (y + 2z)-wertigen Rest darstellt, abgeleitet von einem Polyol mit (y + 2z) oder mehr Hydroxylgruppen durch Entfernung von (y + 2z) Hydroxylgruppen;
- R₁ und R₅: unabhängig voneinander Wasserstoff, Halogen, C₁-C₄Alkyl oder C₁-C₄Alkoxy oder zusammen eine Methylengruppe; und
- R₂, R₃, R₄, R₆, R₇, R₈ und R₉: unabhängig voneinander Wasserstoff, Halogen, C₁-C₄Alkyl oder C₁-C₄Alkoxy; und
- x: eine ganze Zahl von mindestens 3;
- y: eine ganze Zahl von 1 bis (x - 1) und
- z: (x - y) bedeuten.

Die Pulverlacke gemäss der vorliegenden Erfindung zeichnen sich unter anderem durch ein sehr gutes Verlaufsverhalten aus und ergeben ein gehärtetes Material, das eine hohe Vernetzungsdichte, Wetterbeständigkeit und einen hohen Glanz aufweist. Epoxidharze der Formel (I) sind zudem toxikologisch weniger bedenklich als Glycidylverbindungen, wie sie üblicherweise für Pulverlacke eingesetzt werden.

Stellt einer der Reste R₁, R₂, R₃, R₄, R₅, R₆,R₇, R₈ und R₉ in Formel (I) Halogen dar, handelt es sich bevorzugt z. B. um Cl oder Br; stellt einer der genannten Reste C₁-C₄Alkyl oder C₁-C₄Alkoxy dar, handelt es sich z. B. um Methyl, Ethyl, n-Propyl, i-Propyl, n-Butyl, I-Butyl oder t-Butyl oder um die diesen Alkylgruppen entsprechenden Alkoxygruppen.

Vorzugsweise handelt es sich bei den Resten R₁, R₂, R₃, R₄, R₅, R₆,R₇, R₈ und R₉ um C₁-C₄Alkyl oder insbesondere um Wasserstoff.

Die Verbindungen der Formel (I) sind zumindest teilweise bekannt und in bekannter oder hierzu analoger Weise erhältlich.

Verbindungen der Formel (I), worin A einer Gruppe der Formel (II) entspricht, können z. B. aus einem Polyol der Formel B(OH)ₓ erhalten werden, worin x die oben schon genannte Bedeutung hat, indem man die x Hydroxylgruppen des Polyols mit Cydohexen-3-carbonsäure verestert und die Kohlenstoffdoppelbindungen der erhaltenen Polyesterverbindung danach in üblicher Weise, z. B. mit Hilfe einer organischen Persäure, wie z. B. Peressigsäure, epoxidiert.

Eine spezielles bevorzugtes Verfahren zur Herstellung von Verbindungen der Formel (I), worin A einer Gruppe der Formel (II) entspricht, umfasst eine Umesterung eines Cyclohexen-3-carbonsäureesters, insbesondere eines Cyclohexen-3-carbonsäure-C₁-C₄alkylesters, wie Methyl-3-cyclohexencarboxylat, mit einem Polyol der Formel B(OH)ₓ, worin x die oben schon genannte Bedeutung hat, in Gegenwart von LiNH₂ als Umesterungskatalysator unter ständiger Entfernung des aus dem Cyclohexen-3-carbonsäureester freigesetzten Alkohols aus dem Reaktionsgemisch; der eine in üblicher Weise durchgeführte Epoxidierung der Kohlenstoffdoppelbindungen des erhaltenen Umesterungsprodukts, z. B. mit Hilfe einer organischen Persäure, wie z. B. Peressigsäure, folgt. Die Verwendung von LiNH₂ als Katalysator führt unter anderem zu besonders guten Ausbeuten und einer guten Produktreinheit. Das genannte Verfahren ist ebenso auf Epoxidverbindungen der Formel (I) anwendbar, worin A einer Gruppe der Formel (II) entspricht und x gleich 1 oder 2 ist.

Verbindungen der Formel (I), worin A einer Gruppe der Formel (III) entspricht, können z. B. gemäss dem Britischen Patent Nr. 870,696 hergestellt werden, indem man ein Polyol der Formel B(OH)ₓ, worin x die oben schon genannte Bedeutung hat, mit einem Aldehyd der Formel (V) 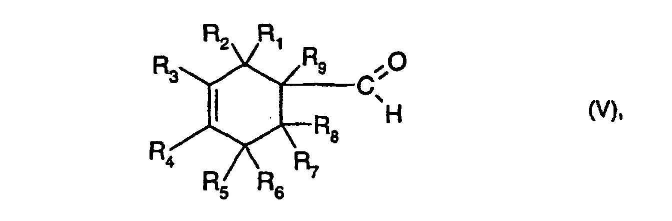 worin R₁ und R₅ sowie R₂, R₃, R₄, R₆, R₇, R₈ und R₉ ebenfalls die oben schon genannte Bedeutung haben, in Gegenwart eines geeigneten Katalysators, wie z. B. p-Toluolsulfonsäure, umsetzt und die Kohlenstoffdoppelbindungen des erhaltenen Produkts in üblicher Weise, z. B. mit Hilfe einer organischen Persäure, epoxidiert

Verbindungen der Formel (I), worin A einer Gruppe der Formel (IV) entspricht, können z. B. gemäss SU-A-1792956 erhalten werden, indem man einen Aldehyd der oben schon genannten Formel (V) in Gegenwart einer Säure, z. B. Phosphor- oder Salpetersäure, trimerisiert, und die Doppelbindungen des hierbei erhaltenen Produkts wieder in üblicher Weise epoxidiert.

Verbindungen der Formel (I), worin A einer Gruppe der Formel (VI) entspricht, sind ebenfalls bekannt, z. B. aus Batog, A. E.; Pet'ko, I. P.; Kozlova, L V.; Pandazi, I. F.; Plast. Massy (1979); (10), p. 9-10 bekannt, wo z. B. eine Verbindung der oben genannten Formel (I) beschrieben ist, worin A der nachstehenden Gruppe entspricht und D einen 4-wertigen Rest, darstellt, der sich von Pentaerythrit durch Entfernung von 4 Hydroxylgruppen ableitet:

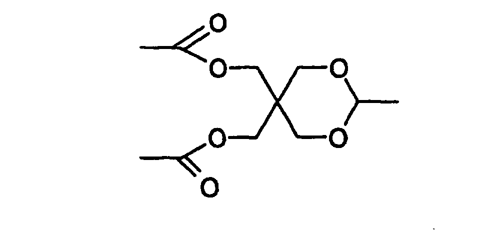

Bevorzugt sind erfindungsgemässe Pulverlackzusammensetzungen, worin A einer Gruppe der Formel (II) entspricht, insbesondere wenn x gleich 3 bis 6 und vorzugsweise gleich 4 ist.

B stellt in Formel (II) vorzugsweise einen Rest dar, abgeleitet von einem aliphatischen Polyol mit 3 bis 20 Kohlenstoffatomen, einem cycloaliphatischen Polyol mit 5 bis 20 Kohlenstoffatomen oder einem gemischt aliphatisch-cycloaliphatischen Polyol mit 7 bis 20 Kohlenstoffatomen.

Besonders bevorzugt leitet sich der Rest B in Formel (II) von 1,3-Dihydroxy-2,2-di(hydroxymethyl)propan (Pentaerythrit) ab.

Weiterhin sind erfindungsgemässe Pulverlackzusammensetzungen bevorzugt, worin A einer Gruppe der Formel (III) entspricht, insbesondere wenn x gleich 3 bis 6 und vorzugsweise gleich 3 ist.

E stellt in Formel (III) ebenso wie D in Formel (VI) vorzugsweise einen Rest dar, abgeleitet von einem aliphatischen Polyol mit 3 bis 20 vorzugsweise 5 oder 6 Kohlenstoffatomen.

Besonders bevorzugt leitet sich der Rest B in Formel (III) von einem Polyol ab, ausgewählt aus Mannit, insbesondere D-Mannit, Sorbit, insbesondere D-Sorbit und Dulcit.

Weiterhin stellen Pulverlackzusammensetzungen, worin A der Gruppe der Formel (IV) entspricht, eine bevorzugte Ausführungsform der Erfindung dar.

Eine andere spezielle Ausführungsform der erfindungsgemässen Pulverlackzusammensetzungen ist dadurch gekennzeichnet, dass sie mindestens eine weitere bei 25°C feste Epoxidverbindung der Formel (I) enthält, worin
- A: einer Gruppe der Formel (II) oder (III) entspricht und
- x: gleich 2 ist.

Für die Reste R₁ bis R₉ gilt bei den Epoxidverbindungen der Formel (I), bei denen x gleich 2 ist, das gleiche wie für die anderen Epoxidverbindungen der Formel (I), ebenso für die Gruppen B und E, soweit dies mit dem Wert x gleich 2 vereinbar ist. Beispiele für geeignete Epoxidverbindungen der Formel (I), worin x gleich 2 ist, umfassen unter anderem: 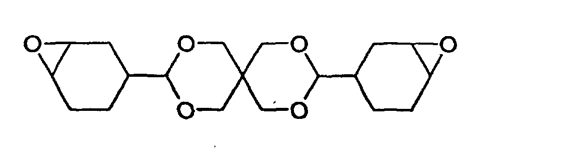 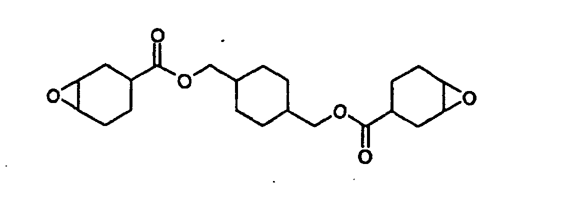 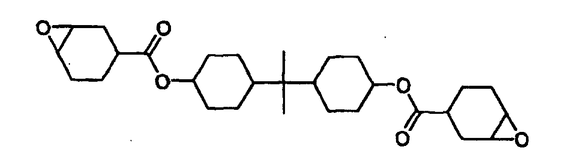 und

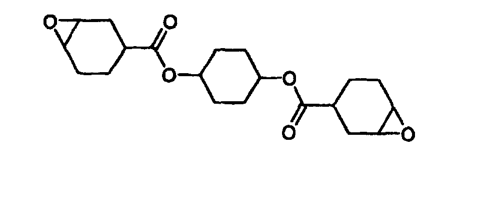

Die Herstellung dieser difunktionellen Epoxidverbindungen kann ebenfalls in der Weise erfolgen, wie sie oben schon für die entsprechenden trifunktionellen und höher funktionellen Verbindungen beschrieben wurde.

Bei diesen Pulverlackzusammensetzungen können die Epoxidverbindungen der Formel (I), worin x mindestens 3 ist, und die Epoxidverbindungen der Formel (I), worin x gleich 2 ist, in einem breit variierbaren molaren Verhältnis vorliegen, z. B. in einem molaren Verhältnis bis zu maximal 1:2, vorzugsweise bis zu maximal 1:1, insbesondere von maximal 1:0,5.

Neben den Epoxidverbindungen der Formel (I) können die erfindungsgemässen Pulverlacke prinzipiell auch bestimmte Mengen einer oder mehrerer anderer Epoxidverbindungen enthalten, z. B. Glycidylester, wie sie in der EP-A-536085, EP-A-770605 oder der EP-A-770650 beschrieben sind. Der Begriff "bestimmte Menge" ist hierbei so zu verstehen, dass durch diese anderen Epoxidverbindungen maximal 60 Prozent, vorzugsweise maximal 5 bis 30 Prozent, der gesamten Epoxidgruppen der erfindungsgemässen Pulverlackzusammensetzungen zur Verfügung gestellt werden. Besonders bevorzugt sind die erfindungsgemässen Pulverlackzusammensetzungen jedoch weitgehend frei von derartigen anderen Epoxidverbindungnen, insbesondere von Glycidylverbindungen, wie TGIC oder Glycidylestern, wie Diglycidylterephthalat, oder die entsprechenden Glycidylmethacrylate oder Copolymere davon. "Weitgehend frei" bedeutet, dass durch TGIC oder Glycidylester maximal 10 Prozent, vorzugsweise maximal 5 Prozent, der gesamten Epoxidgruppen der erfindungsgemässen Pulverlackzusammensetzungen zur Verfügung gestellt werden. Am meisten bevorzugt sind schliesslich erfindungsgemässe Pulverlackzusammensetzungen, die völlig frei von Glycidylverbindungen, insbesondere frei von TGIC und Glycidylestern, sind.

Als Bindemittel für die erfindungsgemässen Pulverlacke eignen sich z. B. Polyester mit freien Carboxylgruppen, die eine Säurezahl von 10 bis 160, vorzugsweise von 10 bis 70, insbesondere von 20 bis 40 mg KOH pro Kilogramm Polyester aufweisen.

Die Polyester sind weiterhin zweckmässigerweise bei Raumtemperatur (15 bis 35°C) fest und weisen z. B. ein Molekulargewicht (Zahlenmittel Mn) von 1000 bis 10000 auf. Das Verhältnis von Mw (Gewichtsmittel des Molekulargewichts) zu Mn liegt bei diesen Polyestem im allgemeinen zwischen 2 und 10. Gut geeignet sind beispielsweise Polyester mit freien Carboxylgruppen, die ein Molekulargewicht (Gewichtsmittel Mw aus GPC-Messung mit Polystyroleichung) von 4000 bis 15000, insbesondere von 6500 bis 11000 und eine Glasumwandlungstemperatur (Tg) von 35 bis 120 °C, vorzugsweise von 50 bis 90 °C aufweisen.

Polyester, wie die erwähnten, sind z. B. in der US-A-3,397,254 oder der EP-A-0 600 546 beschrieben. Für die vorliegende Erfindung geeignete Polyester sind Kondensationsprodukte von difunktionellen, trifunktionellen und/oder polyfunktionellen Alkoholen (Polyolen) mit Dicarbonsäuren und gegebenenfalls trifunktionellen und/oder polyfunktionellen Carbonsäuren, oder mit entsprechenden Carbonsäureanhydriden. Als Polyole kommen beispielsweise Ethylenglycol, Diethylenglycol, die Propylenglycole, Butylenglycol, 1,3-Butandiol, 1,4-Butandiol, Neopentandiol, Isopentylglycol, 1,6-Hexandiol, Glycerin, Hexantriol, Trimethylolethan, Trimethylolpropan, Erythrit, Pentaerythrit, Cyclohexandiol oder 1,4-Dimethylolcyclohexan zur Anwendung. Als Dicarbonsäuren geeignet sind z. B. Isophthalsäure, Terephthalsäure, Phthalsäure, methylsubstituierte Derivate der genannten Säuren, Tetrahydrophthalsäure, Methyltetrahydrophthalsäuren, z. B. 4-Methyltetrahydrophthalsäure, Cyclohexandicarbonsäuren, Bernsteinsäure, Glutarsäure, Adipinsäure, Pimelinsäure, Suberinsäure, Azelainsäure, Sebacinsäure, Dodecandicarbonsäure, Fumarsäure, Maleinsäure oder 4,4'-Diphenyldicarbonsäure usw. Geeignete Tricarbonsäuren sind z. B. aliphatische Tricarbonsäuren, wie 1,2,3-Propantricarbonsäure, aromatische Tricarbonsäuren, wie Trimesinsäure, Trimellitsäure und Hemimellitsäure oder cycloaliphatische Tricarbonsäuren, wie 6-Methyl-cyclohex-4-en-1,2,3-tricarbonsäure. Geeignete Tetracarbonsäuren sind z. B. Pyromellitsäure oder Benzophenon-3,3',4,4'tetracarbonsäure. Sehr häufig basieren insbesondere kommerziell verfügbare Polyester auf Neopentylglycol und/oder Trimethylolpropan als wesentlichen alkoholischen Monomerbestandteilen sowie auf Adipinsäure und/oder Terephthalsäure und/oder Isophthalsäure und/oder Trimellitsäure als wesentlichen Säuremonomerkomponenten.

Als Bindemittel eignen sich weiterhin Poly(meth)acrylate mit Carboxylgruppen, welche sich in bekannter Weise durch Copolymerisation von Acryl- und/oder Methacrylmonomeren herstellen lassen, also beispielsweise von C₁-C₁₂-Alkyl(meth)acrylaten, wie Methyl-, Ethyl-, Propyl-, Butyl-, Pentyl-, Hexyl-, Octyl-, 2-Ethylhexyl-, Decyl- und Dodecyl(meth)acrylaten, wobei C₁-C₄-Alkyl(meth)acrylate bevorzugt sind, oder (Meth)acrylamid mit Acrylsäure und/oder Methacrylsäure und gegebenenfalls zusätzlichen anderen ethylenisch ungesättigten Comonomeren, wie Vinylaromaten, z.B. Styrol, α-Methylstyrol, Vinyltoluol oder auch β-halogenierten Styrolen, erhalten. Die Copolymerisation kann hierbei in bekannter Weise erfolgen. So kann z.B. die Monomeren in geeigneten organischen Lösungsmitteln lösen und in Gegenwart eines geeigneten, in dem Lösungsmittel löslichen Initiators, wie Dicumylperoxid, und eines geeigneten Kettenübertragungsreagenzes, wie Thioglycolsäure, thermisch umsetzen (Lösungspolymerisation) oder das Monomerengemisch zusammen mit einer Lösung des Initiators in einem organischen Lösungsmittel in Wasser suspendieren und polymerisieren oder das Monomerengemisch auch mit Hilfe von Tensiden, z.B. Natriumlaurylsulfat in Wasser emulgieren und in Gegenwart eines wasserlöslichen Polymerisationsinitiators, wie K₂S₂O₈, umsetzen (Emulsionspolymerisation). Danach wird jeweils das fertige Poly(meth)acrylharz aus dem Lösungsmittel oder Wasser in fester Form isoliert. Die Umsetzung kann auch ohne die Verwendung von Lösungsmitteln oder Wasser erfolgen, z.B. gemäss JP-A-Sho 53-140395. Geeignete Poly(meth)acrylharze sind bei Temperaturen im Bereich von Raumtemperatur (15 bis 25°C) fest. Sie weisen im allgemeinen ein Molekulargewicht zwischen 1000 und 50000 (Gewichtsmittel M_{w}) bevorzugt zwischen 5000 und 20000 auf.

Der Tg-Wert (Glasübergangstemperatur) der Poly(meth)acrylate, bestimmt mit DSC (Aufheizgeschwindigkeit 10°C/Minute), beträgt bevorzugt 40 bis 75°C. Die Säurezahl der Harze, angegeben in mg äquivalente KOH pro g (Meth)acrylatharz, beträgt bevorzugt 20 bis 160, bevorzugt 20 bis 80.

In bestimmten Fällen kann es auch vorteilhaft sein, ein Gemisch von Polyestern mit freien Carboxylgruppen und Poly(meth)acrylaten mit freien Carboxylgruppen als Bindemittel einzusetzen.

Die erfindungsgemässen Pulverlackzusammensetzungen enthalten Epoxidverbindungen und Bindemittel bevorzugt in einer Menge, dass das Verhältnis von Epoxidgruppen zu Carboxylgruppen des Bindemittels zwischen 2:1 und 0,5:1 liegt, vorzugsweise zwischen 1,3:1 und 0,7:1. Die erfindungsgemässen Zusammensetzungen können insbesondere einen leichten molaren Überschuss an Epoxidgruppen aufweisen. So beträgt das molare Verhältnis von Epoxidgruppen zu Carboxylgruppen in den Zusammensetzungen vorzugsweise 1,3:1 bis 1:1, z. B. etwa 1,2:1 bis 1,1:1.

Bevorzugt enthalten die erfindungsgemässen Pulverlacke weiterhin einen Katalysator für die Reaktion der Epoxidgruppen mit den Carboxylgruppen. Häufig handelt es sich bei diesen Katalysatoren um ein organisches Amin oder ein Derivat eines Amins, insbesondere um ein tertiäres Amin oder eine stickstoffhaltige heterocyclische Verbindung. Bevorzugte Katalysatoren für die Reaktion von Epoxid- mit Carboxylgruppen sind Phenylimidazol, N-Benzyldimethylamin und 1,8-Diazabicyclo[5,4,0]-7-undecen, gegebenenfalls auf einem Silikatträgermaterial oder Triphenylphosphin, Alkyltriphenyl-phosphoniumhalogenid, Actiron® NXJ-60 (2-Propylimidazol), Actiron®NXJ-60 P (60 Gew.-% von 2-Propylimidazol auf 40 Gew.-% festem Trägermaterial), Beschleuniger® DT 3126 (Alkylammoniumsalz in Polyester). Der Katalysator oder ein Katalysatorgemisch wird bevorzugt in einer Menge zugesetzt, dass die Gelierzeit des Gemisches bei 180°C etwa 70 bis 400 (bestimmt gemäss DIN 55990), bevorzugt 90 bis 300 Sekunden. Im allgemeinen werden hierfür etwa 0,1 bis 10, insbesondere von 0,5 bis 5 Gewichtsprozent des Katalysators benötigt. Kommerziell erhältliche Polyester, die als Bindemittel für die erfindungsgemässen Pulverlackzusammensetzungen eingesetzt werden können, enthalten allerdings in manchen Fällen schon einen gewissen Anteil eines der oben genannten oder eines vergleichbaren Katalysators, der bei dieser Gewichtsprozentangabe zu berücksichtigen ist, wobei die genannten bevorzugten Gelierzeiten als Anhaltspunkt für die noch zuzugebende Menge Katalysator dienen können.

Die erfindungsgemässen Pulverlacke können auch noch weitere in der Lackindustrie übliche Zusatzstoffe enthalten, beispielsweise Lichtschutzmittel, Farbstoffe, Pigmente, z.B. Titandioxidpigment, Entgasungsmittel, z.B. Benzoin, und/oder Verlaufsmittel. Geeignete Verlaufsmittel sind z.B. Polyvinylacetale, wie Polyvinylbutyral, Polyethylenglycol, Polyvinylpyrrolidon, Glycerin, Acrylmischpolymerisate, wie sie z.B. unter den Bezeichnungen Modaflow® oder Acrylron® erhältlich sind.

Erfindungsgemässe Pulverlacke können durch einfaches Mischen der Bestandteile, z.B. in einer Kugelmühle, hergestellt werden. Eine andere und mehr bevorzugte Möglichkeit besteht darin, dass man die Bestandteile zusammen aufschmilzt, vermischt und homogenisiert, vorzugsweise in einer Extrusionsmaschine, wie einem Buss-Kokneter, die Masse abkühlt und zerkleinert. Hierbei erweist es sich als besonders vorteilhaft, dass die erfindungsgemässen Pulverlacke nach der Extrusion entweder gleich zumindest aber nachdem man sie einige Stunden, beispielsweise 24 bis 48 Stunden, stehen gelassen hat, so hart und spröde werden, dass sie ohne weiteres vermahlen werden können. Die Pulverlackmischungen weisen vorzugweise eine Partikelgrösse im Bereich von 0,015 bis 500 µm, insbesondere von 10 bis 75 µm, auf. In manchen Fällen kann es auch zweckmässig sein, zunächst ein Masterbatch aus Teilen des Bindemittels, der Epoxidharze und gegebenenfalls weiterer Komponenten herzustellen, das dann in einem zweiten Schritt mit dem Rest des Bindemittels und den restlichen Bestandteilen zu endgültiger Pulverlackzusammensetzung abgemischt und homogenisiert wird.

Die Pulverlacke werden nach ihrer Applikation auf den zu beschichtenden Gegenstand bei einer Temperatur von mindestens etwa 100°C, z. B. von 150 bis 250°C, gehärtet. Für die Härtung sind im allgemeinen etwa 10 bis 60 Minuten erforderlich. Zur Beschichtung geeignet sind alle Materialien, die bei den zur Härtung erforderlichen Temperaturen stabil sind, insbesondere Keramik und Metall. Das Substrat kann hierbei bereits eine oder mehrere Basislackbeschichtungen aufweisen, die mit dem Pulverlack verträglich sind.

Die Pulverlacke zeigen einen guten Verlauf bei gleichzeitig guten mechanischen Eigenschaften, guter Wetterresistenz und guter Widerstandsfähigkeit gegen Chemikalien.

### Beispiel 1a (Umsetzung von D-Mannit mit 1,2,3,6-Tetrahydrobenzaldehyd):

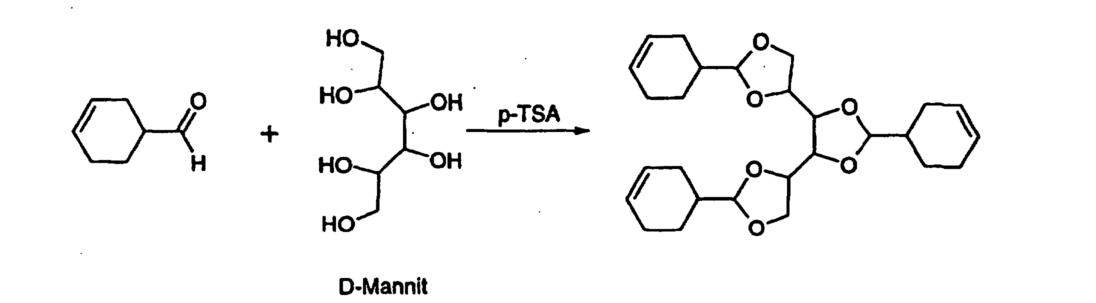

Ein Gemisch von D-Mannit (182.18 g, 1.0 mol), 1,2,3,6-Tetrahydrobenzaldehyd (800 ml, 7.0 mol) and p-Toluolsulfonsäuremonohydrat (1.9 g, 10 mmol, p-TSA) wird unter Rückfluss erhitzt (100°C/200 mbar) und Wasser kontinuierlich azeotrop entfernt. Innerhalb von 2 Stunden wird die theoretisch berechnete Menge Wasser (53 ml) gesammelt und das Gemisch danach auf Raumtemperatur abgekühlt. Dann wird das Gemisch über Dowex (Fluka 44340) filtriert. Die Entfernung des überschüssigen 1,2,3,6-Tetrahydrobenzaldehyds ergibt 460.5 g (100%) des gewünschten Produkts als viskoses Öl.

### Beispiel 1b (Umsetzung von D-Sorbit mit 1,2,3,6-Tetrahydrobenzaldehyd):

In der gleichen Weise wie unter Beispiel 1a beschrieben, werden D-Sorbit (54.50 g, 0.30 mol), 1,2,3,6-Tetrahydrobenzaldehyd (250 ml, 2.2 mol) in Gegenwart von p-Toluolsulfonsäuremonohydrat (0.57 g, 3 mmol) umgesetzt und ergeben 119.0 g (87%) des entsprechenden Produkts, ebenfalls als viskoses Öl.

### Beispiel 1c (Trimerisierung von 1,2,3,6-Tetrahydrobenzaldehyd):

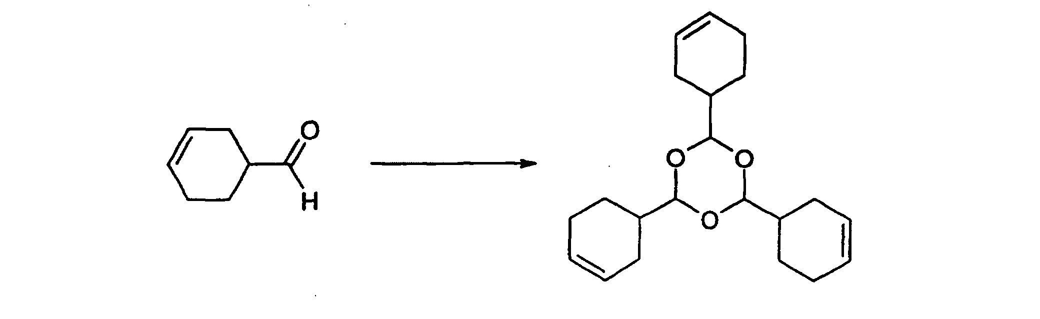

1,2,3,6-Tetrahydrobenzaldehyd (200g, 1.8 mol) wird in einen Reaktor gegeben. Unter starkem Rühren wird ortho-Phosphorsäure tropfenweise zugegeben, wobei die Temperatur auf 20°C gehalten wird. Nach 25 minütiger Reaktion bildet das gesamte Gemisch eine feste Masse und es werden 500 ml Wasser zugegeben. Der feste Rückstand wird fünfmal mit je 800 ml Wasser, danach mit 500 ml NaHCO₃-Lösung (5% in Wasser), dann wieder mit zweimal je 800 ml Wasser und schliesslich zweimal mit je 800 ml Ethanol gewaschen. Der Niederschlag wird abfiltriert und über Nacht bei 60°C getrocknet. Es werden 162.8 g (81%) eines weissen Pulvers vom Schmelzpunkt 170°C erhalten.

### Beispiel 1d (Umsetzung von Pentaerythrit mit 1,2,3,6-Tetrahydrobenzaldehyd):

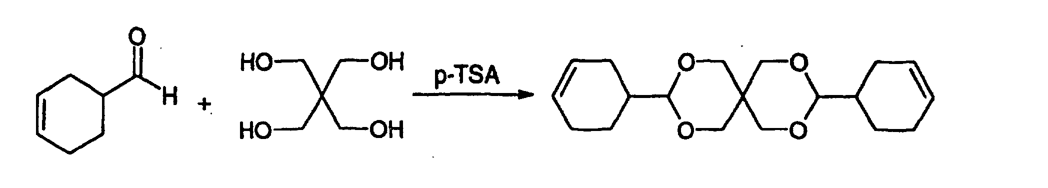

Ein Gemisch von Pentaerythritol (81.76 g, 0.60 mol), 1,2,3,6-Tetrahydrobenzaldehyd (250 ml, 3.5 mol) and p-Toluolsulfonsäuremonohydrat (1.14 g, 6 mmol, p-TSA) wird unter Rückfluss erhitzt (100°C/500 mbar) und Wasser kontinuierlich azeotrop entfernt. Innerhalb von 2,5 Stunden werden 17ml Wasser gesammelt und das Gemisch danach auf Raumtemperatur abgekühlt. Dann wird das Gemisch mit 300 ml Ethylacetat verdünnt, zunächst mit 250 ml NaHCO₃-Lösung (5%-ig in Wasser) und danach zweimal mit 250 ml gesättigter NaCI-Lösung gewaschen. Die organische Schicht wird abgetrennt und über MgSO₄ getrocknet. Nach Entfernung des Lösungsmittels wird das verbleibende Gemisch in 1.5 l kaltes Ethanol geschüttet, und der sich bildende Niederschlag wird abfiltriert, mit Ethanol gewaschen und über Nacht bei 70°C getrocknet. Man erhält 110.3 g (57%) des gewünschten Produkts als gelbes Pulver vom Schmelzpunkt 97°C.

### Beispiel 2a (Epoxidierung des Produkts aus Beispiel 1a):

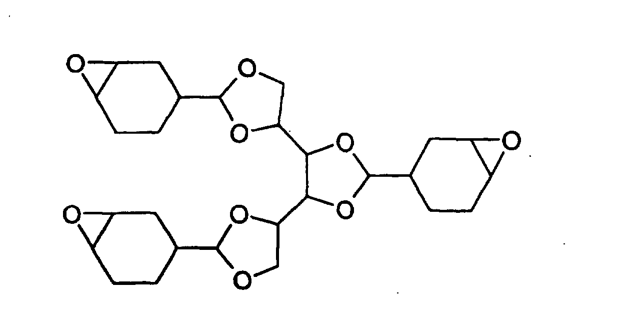

Ein Gemisch aus dem Produkt von Beispiel 1a (114.6 g, 0.25 mol) in 750 ml Dichloromethan wird 10°C gekühlt. Dann wird eine Lösung aus Peressigsäure (39%ig in Essigsäure, 172g, 0.88 mol) und wasserfreiem Natriumacetat (8.79 g, 0.11 mol) wird tropfenweise während einer Stunde zu dem Gemisch gegeben. Während der Zugabe wird die Temperatur unter 30°C gehalten. Danach lässt man das Gemisch 3 Stunden lang bei Raumtemperatur reagieren. Das Gemisch wird mit 500 ml Wasser, 500 ml NaOH-Lösung (1N) und mit 500 ml gesättigter NaCI-lösung gewaschen. Die organische Phase wird abgetrennt, mit Natriumsulfit gerührt und über MgSO₄ getrocknet. Nach Entfernung des Lösungsmittels erhält man 122.8 g (97%) des Produkts als gelbes viskoses Harz (Epoxidzahl: 5.46 val/kg).

### Beispiel 2b (Epoxidierung des Produkts aus Beispiel 1b):

In der gleichen Weise wie unter Beispiel 2a beschrieben, werden das Produkt aus Beispiel 1b (101 g, 0.22 mol), Peressigsäure (39%ig, 151.4 g, 0.78 mol), Natriumacetat (7.72 g, 94 mmol) und Dichloromethan (500 ml) umgesetzt und ergeben 98.0 g (88%) des entsprechenden Endprodukts als viskoses Öl (Epoxidzahl: 5.53 val/kg)

### Beispiel 2c (Epoxidierung des Produkts aus Beispiel 1 c):

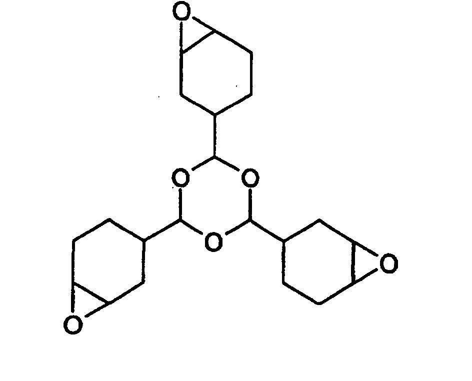

In der gleichen Weise wie unter Beispiel 2a beschrieben, werden das Produkt aus Beispiel 1c (99.1 g, 0.30 mol), Peressigsäure (39%, 207.1 g, 1.06 mol), Natriumacetat (10.61 g, 129 mmol) und Dichloromethan (1000 ml) umgesetzt und ergeben 106.1 g (94%) des entsprechenden Endprodukts als weisses Pulver vom Schmelzpunkt 201°C (Epoxidzahl: 7.55 val/kg.)

### Beispiel 2e (Epoxidierung einer Mischung der Produkte aus den Beispielen 1b und 1c im molaren Verhältnis 1:1):

In der gleichen Weise wie unter Beispiel 2a beschrieben, werden das Produkt aus Beispiel 1b (114.7 g, 0.25 mol) und das Produkt aus Beispiel 1c (82.6 g, 0.25 mol), Peressigsäure (39%, 343.8 g, 1.76 mol), Natriumacetat (17.36 g, 212 mmol) und Dichloromethan (1000 ml) umgesetzt und ergeben 215.8 g (97%) des entsprechenden Gemisches von Epoxidverbindungen der Formel (I) als gelbes Pulver (Epoxidzahl: 6.08 val/kg)

### Beispiel 2f (Epoxidierung einer Mischung der Produkte aus den Beispielen 1a und 1c im molaren Verhältnis von 42:58):

In der gleichen Weise wie unter Beispiel 2a beschrieben, werden das Produkt aus Beispiel 1a (33.0 g, 72 mmol) und das Produkt aus Beispiel 1c (33.0 g, 100 mmol), Peressigsäure (39%, 118.0 g, 607 mmol), Natriumacetat (6.03 g, 73 mmol) und Dichloromethan (500 ml) umgesetzt und ergeben 69.1 g (97%) des entsprechenden Gemisches von Epoxidverbindungen der Formel (I) als weisses Pulver (Epoxidzahl: 6.55 val/kg)

### Beispiel 2g (Epoxidierung einer Mischung der Produkte aus den Beispielen 1c und 1d im molaren Verhältnis von ca. 1:1):

In der gleichen Weise wie unter Beispiel 2a beschrieben, werden das Produkt aus Beispiel 1c (50.0 g, 156 mmol) und das Produkt aus Beispiel 1d (50.0 g, 151 mmol), Peressigsäure (39%, 196.1 g, 1.0 mol), Natriumacetat (10.0 g, 122 mmol) und Dichloromethan (800 ml) umgesetzt und ergeben 98.4 g (87%) des entsprechenden Gemisches von Epoxidverbindungen der Formel (I) als weisses Pulver (Epoxidzahl: 5.72 val/kg)

### Beispiel 3:

Mit einem Extruder (Laborextruder der Firma PRISM, The Old Stables, England) wird die in der nachfolgenden Tabelle 3/1 angegebene Pulverlackzusammensetzung homogenisiert. Das abgekühlten Extrudat wird zum fertigen Pulverlack mit einer Partikelgrösse von etwa 40 Micrometer vermahlen.

**Tabelle 3/1:**

| Pulverlackformulierung | |
|---|---|
| Formulierung | A [g] |
| Uralac P 3485¹⁾ | 60.00 |
| Epoxidverbindung gemäss Beispiel 2a | 5.80 |
| Benzoin | 0.20 |
| Acrylron²⁾ | 1.00 |
| TiO₂ [Cronos 2160] | 33.00 |

| | |
|---|---|
| ¹⁾ Polyester auf Basis von Terephthalsäure, Isophthalsäure und Neopentylglycol mit einer Säurezahl von 28 und einer Glasübergangstemperatur Tg von 71 °C; | |
| ²⁾ Acrylmischpolymerisat als Verlaufsmittel. | |

Mit Hilfe einer elektrostatischen Sprühpistole wird die Pulverlackzusammensetzung auf ein Q-Paneel als Substrat aufgetragen. Danach wird das beschichtete Paneel in einen Ofen gegeben, um den Pulverlack zu schmelzen und auszuhärten. Die Gelierzeit, die Härtungstemperatur und Härtungsdauer sowie die Dicke der erhaltenen Pulverlackbeschichtung sind in der folgenden Tabelle 3/2 angegeben, ebenso wie wichtige lacktechnische Eigenschaften der jeweils erhaltenen Beschichtungen.

**Tabelle 3/2:**

| Eigenschaft | A |
|---|---|
| Gelierzeit 180°C [s] | 210 |
| Aushärtung | 15 min. / 200°C |
| Schichtdicke [mm] | 55 |
| Substrat | Q-Panel |
| Glanz 60° | 94 |
| Glanz 20° | 84 |
| Gelbwert Yi | 4.8 |
| Verlauf [Note]³⁾ | 12 |
| Acetontest⁵⁾, 1 min. [Note] | 3 |

| | |
|---|---|
| ³⁾ Empirische Skala von 0 (sehr gut) bis 18 (Orangenhaut) | |
| ⁵⁾ Nach DIN 53320 Die Probe wird 1 min in Aceton gehalten. Das Ergebnis wird gemäss der folgenden fünfteiligen Skala beurteilt: 0 = unverändert; 1 = bremsend, nicht mit dem fingernagel kratzbar; 2 = schwer kratzbar, evtl. Watte gefärbt; 3 = erweicht, leicht kratzbar; 4 = beginnende Ab- oder Auflösung; 5 = vollständige Auflösung. | |

### Beispiel 4:

Mit einem Extruder (Laborextruder der Firma PRISM, The Old Stables, England) werden die in den nachfolgenden Tabelle 4/1 angegebenen Pulverlackzusammensetzungen homogenisiert. Die Gesamtmenge der Pulverlackzusammensetzung beträgt jeweils ca. 100 bis 200 Gramm. Die abgekühlten Extrudate werden zum fertigen Pulverlack mit einer Partikelgrösse von etwa 40 mm vermahlen.

**Tabelle 4/1:**

| Pulverlackformulierungen | | | | |
|---|---|---|---|---|
| Formulierung | B [g] | C [g] | D [g] | E [g] |
| Uralac P 3485¹⁾ | 59.05 | 58.11 | 58.71 | 59.17 |
| DGT⁶⁾ | --- | 1.80 | 4.37 | 4.41 |
| Epoxidverbindung gemäss Beispiel 2b | 5.92 | 4.56 | 1.38 | 1.39 |
| DT 3126⁷⁾ | --- | 0.50 | 0.50 | --- |
| Benzoin | 0.20 | 0.20 | 0.20 | 0.20 |
| Acrylron²⁾ | 1.50 | 1.50 | 1.50 | 1.50 |
| TiO₂ [Cronos 2160] | 33.33 | 33.33 | 33.33 | 33.33 |

| | | | | |
|---|---|---|---|---|
| ⁶⁾ Diglycidylterephthalat | | | | |
| ⁷⁾ Alkylammoniumsalz in Polyester | | | | |

Man findet die in der folgenden Tabelle 4/2 angegebenen Eigenschaften der Beschichtungen.

**Tabelle 4/2:**

| Eigenschaft | B | C | D | E |
|---|---|---|---|---|
| Gelierzeit @180°C [s] | 165 | 265 | 400 | 530 |
| Aushärtung | 15 min. / 180°C | 15 min. / 200°C | 15 min./200°C | 15 min. / 200°C |
| Schichtdicke [mm] | 54 | 55 | 56 | 55 |
| Substrat | Q-Panel | Q-Panel | Q-Panel | Q-Panel |
| Glanz 60° | 95 | 95 | 96 | 96 |
| Glanz 20° | 84 | 84 | 88 | 88 |
| Gelbwert Yi | 2.7 | 4.8 | 1.7 | 0.3 |
| Verlauf³⁾ [Note] | 10 | 10 | 10 | 6 |
| Acetontest⁵⁾, 1 min. [Note] | 3 | 3 | 3 | 4 |

### Beispiel 5:

Die in Tabelle 5/1 angegebenen Pulverlacke werden gemäss Beispiel 4 hergestellt.

**Tabelle 5/1:**

| Pulverlackformulierungen | | | |
|---|---|---|---|
| Formulierung | F [g] | G [g] | H [g] |
| Uralac P 3485¹⁾ | 58.38 | 59.91 | 59.91 |
| Epoxidverbindung gemäss Beispiel 2c | 4.59 | 4.89 | 4.89 |
| DT 3126⁷⁾ | 2.00 | --- | --- |
| Benzoin | 0.20 | 0.20 | 0.20 |
| Crylcoat 164⁹⁾ | --- | 1.00 | --- |
| Acrylron²⁾ | 1.50 | 1.00 | 1.00 |
| TiO₂ [Cronos 2160] | 33.33 | 33.00 | 33.00 |

| | | | |
|---|---|---|---|
| ⁹⁾ Alkyltriphenylphosphoniumbromid in Polyester | | | |

Man findet die in der folgenden Tabelle 5/2 angegebenen Eigenschaften der Beschichtungen.

**Tabelle 5/2:**

| Eigenschaft | F | G | H |
|---|---|---|---|
| Gelierzeit @180°C [s] | 150 | 160 s | 90 |
| Aushärtung | 15 min./180°C | 15 min./200°C | 15 min./200°C |
| Schichtdicke [mm] | 53 | 48 | 51 |
| Substrat | Q-Panel | Q-Panel | Q-Panel |
| Glanz 60° | --- | 95 | 95 |
| Glanz 20° | --- | 83 | 71 |
| Gelbwert Yi | --- | 2.4 | 0.0 |
| Verlauf [Note]³⁾ | 8 | 10 | 10 |
| Acetontest⁵⁾, 1 min. [Note] | 3 | 3 | 3 |

### Beispiel 6:

Der in Tabelle 6/1 angegebene Pulverlack wird gemäss Beispiel 4 hergestellt.

**Tabelle 6/1:**

| Pulverlackformulierung | |
|---|---|
| Formulierung | \| [g] |
| Uralac P 3485¹⁾ | 59.54 |
| Epoxidverbindung gemäss Beispiel 2e | 5.43 |
| Benzoin | 0.20 |
| Acrylron | 1.50 |
| TiO₂ [Cronos 2160] | 33.33 |

Man findet die in der folgenden Tabelle 6/2 angegebenen Eigenschaften für die entsprechende Beschichtung.

**Tabelle 6/2:**

| Eigenschaft | \| |
|---|---|
| Gelierzeit 180°C [s] | 285 |
| Aushärtung | 15 min. / 180°C |
| Schichtdicke [mm] | 86 |
| Substrat | Q-Panel |
| Glanz 60° | 91 |
| Glanz 20° | 75 |
| Gelbwert Yi | 3.3 |
| Verlauf [Note]³⁾ | 10-12 |
| Acetontest,⁵⁾ 1 min. [Note] | 2 |

### Beispiel 7:

Die in Tabelle 7/1 angegebenen Pulverlacke werden gemäss Beispiel 4 hergestellt.

**Tabelle 7/1:**

| Pulverlackformulierungen | | |
|---|---|---|
| Formulierung | J [g] | K [g] |
| Uralac P 3485¹⁾ | 60.66 | 59.79 |
| Epoxidharz gemäss | 5.14 | 6.01 |
| Beispiel 2f | | |
| Benzoin | 0.20 | 0.20 |
| Acrylron²⁾ | 1.00 | 1.00 |
| TiO₂ [Cronos 2160] | 33.00 | 33.33 |

Man findet die in der folgenden Tabelle 7/2 angegebenen Eigenschaften für die entsprechende Beschichtung.

**Tabelle 7/2:**

| Eigenschaft | J | K |
|---|---|---|
| Gelierzeit @180°C [s] | 200 | 200 |
| Aushärtung | 15 min. / 200°C | 15 min. / 200°C |
| Schichtdicke [mm] | 45 | 89 |
| Substrat | Q-Panel | Q-Panel |
| Glanz 60° | 95 | 96 |
| Glanz 20° | 82 | 77 |
| Gelbwert Yi | 2.0 | 7.9 |
| Verlauf³⁾[Note] | 10 | 6-8 |
| Acetontest⁵⁾, 1 min. [Note] | 3 | 3 |

### Beispiel 8:

Die in Tabelle 8/1 angegebenen Pulverlacke werden gemäss Beispiel 4 hergestellt.

**Tabelle 8/1:**

| Pulverlackformulierungen | | |
|---|---|---|
| Formulierung | L [g] | M [g] |
| Uralac P 3485¹⁾ | 91.27 | 60.78 |
| Epoxidverbindung gemäss Beispiel 2e | 7.53 | 5.02 |
| Benzoin | 0.20 | 0.20 |
| Acrylron²⁾ | 1.00 | 1.00 |
| TiO₂ [Cronos 2160] | --- | 33.00 |

Man findet die in der folgenden Tabelle 8/2 angegebenen Eigenschaften für die entsprechenden Beschichtungen.

**Tabelle 8/2:**

| | L | M |
|---|---|---|
| Gelierzeit @180°C [s] | 180 | 180 |
| Aushärtung | 15 min. / 200°C | 15 min. / 200°C |
| Schichtdicke [mm] | 55 | 55 |
| Substrat | Q-Panel | Q-Panel |
| Glanz 60° | 108 | 96 |
| Glanz 20° | 76 | 81 |
| Gelbwert Yi | --- | 6.8 |
| Verlauf³⁾ [Note] | 2 | 6-8 |
| Acetontest⁵⁾, 1 min. [Note] | 3 | 3 |

### Beispiel 9: Herstellung einer Epoxidverbindung der nachstehenden Formel:

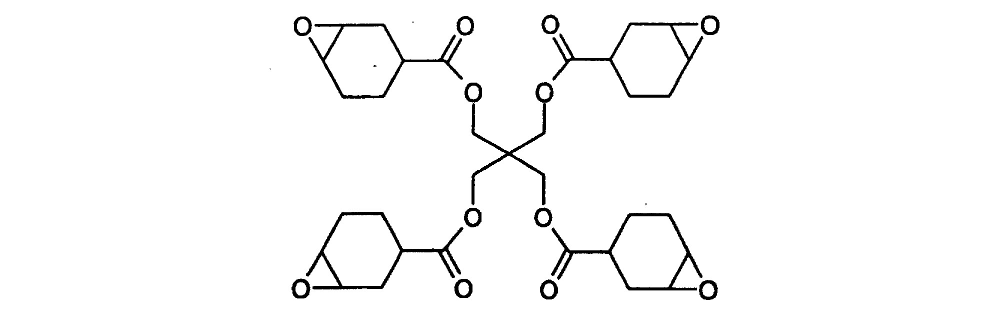

### Stufe A)

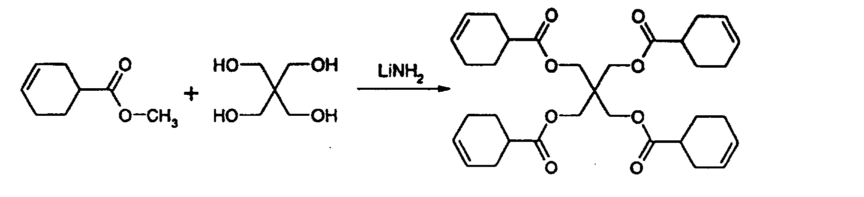

In ein gut isoliertes Reaktionsgefäss, ausgestattet mit einem Thermometer, einem mechanischem Rührer und einer Destillationsbrücke werden 100 ml Xylol (purissimum, aufbewahrt über einem 4Å-Molekularsieb, Wassergehalt < 0,02%), 146,3 g (1,04 Mol) Methyl-3-cyclohexencarboxylat und 27,1 g (0,20 mol) Pentaerythrit gegeben. Die erhaltene Suspension wird unter Stickstoff und Rühren 30 Minuten lang auf eine Temperatur von 145 bis 150°C erhitzt, wobei allmählich weiteres Xylol in das Reaktionsgefäss mit einer Geschwindigkeit zugegeben wird, dass Xylol im gleichen Umfang aus dem Gefäss abdestilliert. Sodann werden 0,23 g (0,01 mol) LiNH₂ zugegeben. Nach etwa 30 Minuten beginnt Methanol abzudestillieren. Die gesamte Destillationszeit beträgt etwa 6 Stunden, wobei 150 ml Xylol zugegeben werden. Danach wird das Reaktionsgefäss auf Raumtemperatur abgekühlt. Das Reaktionsgemisch wird mit 200 ml Toluol verdünnt und mit 200 ml Wasser gewaschen. Die organische Phase wird über MgSO₄ getrocknet und filtriert. Danach werden das Lösungsmittel und überschüssiges Methyl-3-cyclohexencarboxylat mit Hilfe eines Rotationsverdampfers entfernt (120 °C/5 mbar). Es werden 110 g (97% Ausbeute) des Reaktionsproduktes in Form einer farblosen, viskosen Flüssigkeit erhalten, die beim Stehenlassen kristallisiert. Der Schmelzpunkt des Kristallisats beträgt 65°C.

### Stufe B)

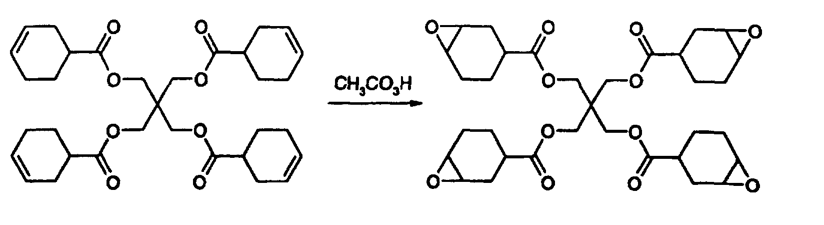

Eine Mischung aus 90,0 g (0,16 Mol) des gemäss Schritt A erhaltenen Reaktionsprodukts in 700 ml Dichlormethan wird auf eine Temperatur von 10°C gekühlt und eine Suspension von 148 g (0,76 Mol, 39%-ig in Essigsäure) Peressigsäure und 7,3 g (0,088 Mol) wasserfreiem Natriumacetat tropfenweise während eines Zeitraums von ca. 45 Minuten zugegeben. Während der Zugabe wird die Temperatur unterhalb von 30°C gehalten. Dann wird die Lösung ca. 3 Stunden lang bei Raumtemperatur (25-30°C) weiter reagieren lassen. Das erhaltene Reaktionsgemisch wird zweimal mit 200 ml Wasser, danach zweimal mit 200 ml einer 5%-igen NaHCO₃ Lösung und schliesslich wiederum zweimal mit 200 ml Wasser gewaschen. Die organische Phase wird danach mit Natriumsulfit verrührt, bis ein Peroxidtest negativ verläuft, und dann über MgSO₄ getrocknet. Nach Entfernung des Lösungsmittels erhält man eine farblose, viskose Flüssigkeit, die beim Stehenlassen langsam kristallisiert. Eine Umkristallisation aus 200 ml MeOH ergibt 80 g (80% Ausbeute) des gewünschten Produkts in Form eines weissen, kristallinen Pulvers (Epoxidwert: 6,1 eq/kg; Schmelzpunkt: 95°C).

### Beispiel 10:

Die in Tabelle 10/1 angegebenen Pulverlacke werden gemäss Beispiel 4 hergestellt.

**Tabelle 10/1:**

| Pulverlackformulierungen | | | | |
|---|---|---|---|---|
| Formulierung | N [g] | O [g] | P [g] | Q [g] |
| Uralac P 3485¹⁾ | 56.26 | 58.89 | 58.80 | 58.82 |
| DGT⁶⁾ | --- | 2.74 | --- | --- |
| HHDGP¹⁰⁾ | --- | --- | --- | 1.12 |
| HHDGT¹¹⁾ | --- | --- | 1.14 | ---- |
| Epoxidverbindung gemäss Beispiel 9 | 5.71 | 2.84 | 4.53 | 4.53 |
| DT 3126⁷⁾ | --- | 0.50 | 0.50 | 0.50 |
| Benzoin | 0.20 | 0.20 | 0.20 | 0.20 |
| Acrylron²⁾ | 1.50 | 1.50 | 1.50 | 1.50 |
| TiO₂ [Cronos 2160] | 33.33 | 33.33 | 33.33 | 33.33 |

| | | | | |
|---|---|---|---|---|
| ¹⁰⁾ Hexahydrodiglycidylphthalat (Araldit PY 284) | | | | |
| ¹¹⁾ Hexahydrodiglycidylterephthalat | | | | |

Man findet die in der folgenden Tabelle 10/2 angegebenen Eigenschaften für die entsprechenden Beschichtungen

**Tabelle 10/2:**

| | N | O | P | Q |
|---|---|---|---|---|
| Gelierzeit @180°C [s] | 190 | 280 | 195 | 195 |
| Aushärtung | 15 min. / | 15 min. / | 15 min. / | 15 min. / |
| | 180°C | 180°C | 180°C | 180°C |
| Schichtdicke [mm] | 62 | 48 | 58 | 60 |
| Substrat | Q-Panel | Q-Panel | Q-Panel | Q-Panel |
| Glanz 60° | 95 | 95 | 96 | 95 |
| Glanz 20° | 86 | 83 | 88 | 85 |
| Gelbwert Yi | 0.4 | -1.3 | -0.4 | -0.6 |
| Verlauf³⁾[Note] | 10 | 10 | 10 | 11 |
| Schlag, rückseitig⁴⁾ [kg cm] | 100 | >160 | 140 | 120 |
| Schlag, vorne⁴⁾ [kg cm] | 160 | >160 | 160 | 160 |
| Acetontest⁵⁾, 1 min. [Note] | 3 | 3 | 3 | 3 |

| | | | | |
|---|---|---|---|---|
| ⁴⁾ Die Schlagverformung wird bestimmt, indem man einen Stempel mit einem Gewicht von 2 kg, an dessen Unterseite sich eine Kugel von 20 mm Durchmesser befindet, mit der Unterseite voraus aus bestimmter Höhe von hinten (rückseitig) oder von vorne auf die beschichtete Fläche fallen lässt. Der angegebenen Wert ist das Produkt aus dem Gewicht des Stempels in kg und der Versuchshöhe in cm, bei der noch keine Beschädigung der Beschichtung feststellbar ist. | | | | |

### Beispiel 11:

Die in Tabelle 11/1 angegebenen Pulverlacke werden gemäss Beispiel 4 hergestellt.

**Tabelle 11/1:**

| Pulverlackformulierungen | | | | | |
|---|---|---|---|---|---|
| Formulierung | R[g] | S[g] | T[g] | U[g] | V[g] |
| Uralac P 3485¹⁾ | 58.53 | 58.21 | 58.60 | 57.99 | 58.49 |
| Epoxidverbindung gemäss Beispiel 9 | 4.51 | 4.84 | 4.51 | 5.12 | 4.78 |
| Epoxidverbindung¹²⁾ | 1.43 | 1.42 | - | - | - |
| Epoxidverbindung¹³⁾ | - | - | 1.36 | 1.36 | - |
| Epoxidverbindung¹⁴⁾ | - | - | - | - | 1.20 |
| DT 3126⁷⁾ | 0.50 | 0.50 | 0.50 | 0.50 | 0.50 |
| Benzoin | 0.20 | 0.20 | 0.20 | 0.20 | 0.20 |
| Acrylron²⁾ | 1.50 | 1.50 | 1.50 | 1.50 | 1.50 |
| TiO₂ [Cronos 2160] | 33.33 | 33.33 | 33.33 | 33.33 | 33.33 |

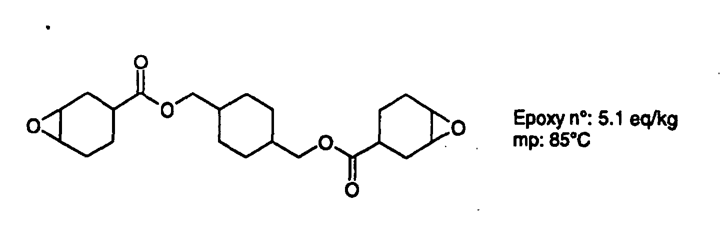

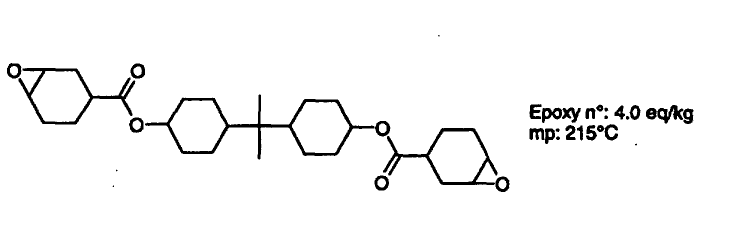

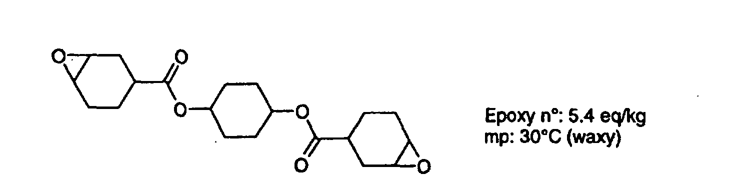

Die obigen Epoxidverbindungen 12), 13) und 14) werden nach dem gleichen Herstellungsverfahren wie im Beispiel 9 gewonnen.

Man findet die in der folgenden Tabelle 11/2 angegebenen Eigenschaften für die entsprechenden Beschichtungen.

**Tabelle 11/2:**

| | R | S | T | U | V |
|---|---|---|---|---|---|
| Gelierzeit @180°C [s] | 240 | 230 | 210 | 215 | 215 |
| Aushärtung | 15 min. | 15 min. | 15 min. | 15 min. | 15 min. |
| | 180°C | 200°C | 200°C | 200°C | 200°C |
| Schichtdicke [mm] | 48 | 47 | 72 | 47 | 47 |
| Substrat | Q-Panel | Q-Panel | Q-Panel | Q-Panel | Q-Panel |
| Glanz 60° | 91 | 94 | 94 | 94 | 95 |
| Glanz 20° | 86 | 84 | 87 | 77 | 82 |
| Gelbwert Yi | -2.3 | -2.1 | 2.0 | -2.8 | -1.9 |
| Verlauf³⁾ [Note] | 10 | 10 | 10 | 11 | 9 |
| Schlag, rückseitig⁴⁾ [kg cm] | >160 | >160 | >160 | >160 | >160 |
| Schlag, vorne⁴⁾ [kg cm] | >160 | >160 | >160 | >160 | >160 |
| Acetontest⁵⁾, 1 min. [Note] | 3 | 3 | 3 | 3 | 3 |

### Beispiel 12:

Die in Tabelle 12/1 angegebenen Pulverlacke werden gemäss Beispiel 4 hergestellt.

**Tabelle 12/1:**

| Pulverlackformulierungen | | | |
|---|---|---|---|
| Formulierung | X [g] | Y [g] | Z [g] |
| Uralac P 3485 | 58.56 | 57.86 | 57.55 |
| Epoxidverbindung, hergestellt gemäss Beispiel 2b | 2.90 | 2.15 | 5.13 |
| (5,60 eq/kg) | | | |
| Epoxidverbindung¹²⁾ | 3.01 | 4.46 | - |
| Epoxidverbindung¹³⁾ | - | - | 1.80 |
| DT 3126⁷⁾ | 0.50 | 0.50 | 0.50 |
| Benzoin | 0.20 | 0.20 | 0.20 |
| Acrylron²⁾ | 1.50 | 1.50 | 1.50 |
| TiO₂ [Cronos 2160] | 33.33 | 33.33 | 33.33 |

| | | | |
|---|---|---|---|
| ^{12);13);7);2)} siehe entsprechende vorstehende Definitionen. | | | |

Man findet die in der folgenden Tabelle 12/2 angegebenen Eigenschaften für die entsprechenden Beschichtungen

**Tabelle 12/2:**

| | X | Y | Z |
|---|---|---|---|
| Gelierzeit @ 180°C [s] | 245 s | 410 s | 220 s |
| Aushärtung | 15 min. / 180°C | 15 min. / 180°C | 15 min. / 180°C |
| Schichtdicke [mm] | 60 | 59 | 69 |
| Substrat | Q-Panel | Q-Panel | Q-Panel |
| Glanz 60° | 95 | 94 | 95 |
| Glanz 20° | 88 | 86 | 86 |
| Gelbwert Yi | 4.5 | 3.3 | 5.1 |
| Verlauf³⁾ [Note] | 10 | 10 | 10 |
| Acetontest⁵⁾, 1 min. [Note] | 3 | 3 | 3 |

### Beispiel 13:

Eine erfindungsgemässe Pulverklarlackzusammensetzung (W) sowie drei zu Vergleichszwecken angeführte Pulverklarlackzusammensetzungen (W1, W2, W3), jeweils auf Basis von Uralac 3489¹⁴⁾ und der in der nachfolgenden Tabelle 13/1 angegebenen Epoxidverbindungen (Molverhältnis der COOH-Gruppen des Polyesters zu den Epoxidgruppen der Epoxidverbindung jeweils 0.95 zu 1) sowie 0.2 Gewichtsprozenten Benzoin und 1.5 Gewichtsprozenten Acrylon (alle Zusammensetzungen ohne Härtungsbeschleuniger) werden durch zweimalige Extrusion mit Hilfe eines Laborextruders der Firma PRISM, The Old Stables, England, (T1=30°C/T2=80°C) homogenisiert. Bestimmt werden jeweils die Gelierzeit bei 180°C; der Prozentsatz geliertes Material (Gelanteil) in der gehärteten Zusammensetzung nach einer Härtung von 15 min bei 200°C, der Tg-Wert der gehärteten Zusammensetzung sowie die Viskosität der gehärteten Systeme bei 180°C. Die Werte sind ebenfalls in Tabelle 13/1 angegeben.

**Tabelle 13/1:**

| Pulverlackformulierungen | | | | | |
|---|---|---|---|---|---|
| Formulierung | Epoxidverbindung (Gew.-%) | Gelierzeit@ 180°C [s] | Gelanteil¹⁸⁾[Gew.-%] | Tg nach Härtung, Beginn [°C] | Viskosität¹⁹⁾ [Pa.s] |
| W gemäss Erfindung | Epoxid gemäss Beispiel 9 (7.8%) | 375 | 93.5 | 76 | 10700 |
| Vergleich W1 | PT 910 ¹⁵⁾(7.2%) | 450 | 80.4 | 72 | 510 |
| Vergleich W2 | XB 912 ¹⁶⁾(7.2%) | 390 | 89.0 | 73 | 2100 |
| Vergleich W3 | PT 810 ¹⁷⁾(5.2%) | 210 | 95.2 | 76 | 9050 |

| | | | | | |
|---|---|---|---|---|---|
| ¹⁴⁾ Uralac P3489 ist ein Polyester auf Basis von Terephthalsäure, Isophthalsäure und Neopentylglycol mit einem Säurewert von 28 mg KOH/g und einem Tg von 70°C. ¹⁵⁾ Araldit PT910 ist eine feste Mischung aus 75 Gew.-% Diglycidylterephthalat und 25 Gew.-% Triglycidyltrimellitat gemäss US-A-5457168. | | | | | |
| ¹⁶⁾ XB 912 ist eine eine feste Mischung aus 60 Gew.-% Diglycidylterephthalat und 40 Gew.-% Triglycidyltrimellitat gemäss US-A-5457168. | | | | | |
| ¹⁷⁾ Araldit PT810 = Triglycidylisocyanurat | | | | | |
| ¹⁸⁾ Der Gelanteil wird folgendermassen bestimmt: Eine 1 Gramm schwere Probe eines 50µm dicken Films der 15 min bei 200°C gehärteten Pulverlackzusammensetzung wird dreimal je 2 Stunden mit jeweils 50 ml Aceton extrahiert. Der angegebene Wert entspricht dem hierbei verbleibenden ungelösten Rückstand, angegeben in Gewichtsprozent. | | | | | |
| ¹⁹⁾ Die Messung der (dynamischen) Viskosität wird mit einem Rheometricsmessgerät bestimmt. Die Probe wird zwischen zwei parallelen Platten (Durchmesser 50mm) einer oszillierenden Scherung (1Hz; Scherung (strain) 15%). Es wird die Viskosität bei 180°C in Abhängigkeit von der Zeit bestimmt. Die in der Tabelle angegebenen Werte entsprechen der Viskosität des gehärteten Systems. | | | | | |

Der gegenüber den Vergleichszusammensetzungen auf Basis von Araldit PT910 (Vergleich W1) und XB 912 (Vergleich W2) signifikant gestiegene Gelanteil der erfindungsgemässen Pulverlackzusammensetzung (W) ist ein deutlicher Hinweis auf die bei gleichen Härtungsbedingungen (15 min/200°C) überraschend angestiegene Vernetzungsdichte in gehärtetem Material auf Basis der erfindungsgemässen Zusammensetzung. Die bei gleicher Härtungszeit gestiegene Vernetzungsdichte macht auch die höhere Reaktivität der erfindungsgemässen Systeme deutlich, die mit Zusammensetzungen auf Basis von Araldit PT810 vergleichbar ist (Vergleich W3). Ebenso zeigt auch die gegenüber allen drei Vergleichssystemen (W1, W2 und W3) signifikant gestiegene Viskosität des erfindungsgemässen Systems nach der Härtung die vergleichsweise hohe Vernetzungsdichte und Reaktivität der erfindungsgemässen Pulverlackzusammensetzungen.

## Patentansprüche

1. Pulverlackzusammensetzung enthaltend ein Bindemittel, das ausgewählt ist aus einem Carboxylgruppen enthaltenden Polyester, einem Carboxylgruppen enthaltenden Poly(meth)-acrylat und Gemischen der genannten Substanzen, und eine oder mehrere Epoxidverbindungen, **dadurch gekennzeichnet, dass** die Epoxidverbindungen mindestens eine bei 25°C feste Verbindung der Formel (I) umfassen: 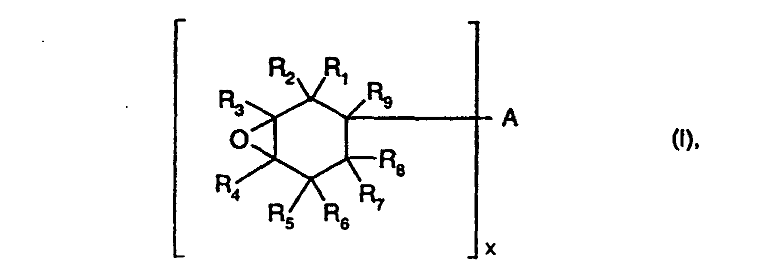 worin
A einer Gruppe der Formel (II), (III), (IV) oder (VI) entspricht 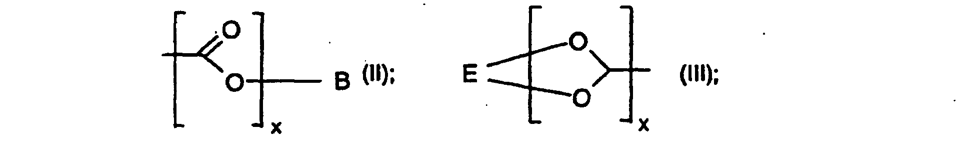 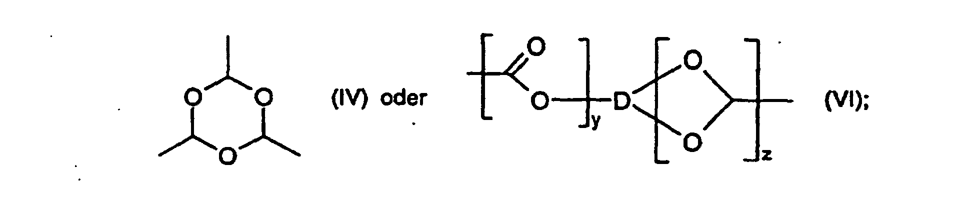
worin
B einen x-wertigen organischen Rest darstellt, abgeleitet von einem Polyol mit x oder mehr Hydroxylgruppen durch Entfernung von x Hydroxylgruppen;
E einen (2x)-wertigen organischen Rest darstellt, abgeleitet von einem Polyol mit (2x) oder mehr Hydroxylgruppen durch Entfernung von (2x) Hydroxylgruppen; und
D einen (y + 2z)-wertigen Rest darstellt, abgeleitet von einem Polyol mit (y + 2z) oder mehr Hydroxylgruppen durch Entfernung von (y + 2z) Hydroxylgruppen;
R₁ und R₅ unabhängig voneinander Wasserstoff, Halogen, C₁-C₄Alkyl oder C₁-C₄Alkoxy oder zusammen eine Methylengruppe; und
R₂, R₃, R₄, R₆, R₇, R₈ und R₉ unabhängig voneinander Wasserstoff, Halogen, C₁-C₄Alkyl oder C₁-C₄Alkoxy; und
x eine ganze Zahl von mindestens 3;
y eine ganze Zahl von 1 bis (x - 1) und
z (x - y) bedeuten.

2. Pulverlackzusammensetzung nach Anspruch 1, **dadurch gekennzeichnet, dass**
A einer Gruppe der Formel (II) entspricht.

3. Pulverlackzusammensetzung nach Anspruch 2 **dadurch gekennzeichnet, dass**
x 3 bis 6, vorzugsweise 4 bedeutet.

4. Pulverlackzusammensetzung nach Anspruch 3, **dadurch gekennzeichnet, dass**
B einen Rest darstellt, abgeleitet von einem aliphatischen Polyol mit 3 bis 20 Kohlenstoffatomen, einem cycloaliphatischen Polyol mit 5 bis 20 Kohlenstoff-atomen oder einem gemischt aliphatisch-cycloaliphatischen Polyol mit 7 bis 20 Kohlenstoffatomen.

5. Pulverlackzusammensetzung nach einem der Ansprüche 1 bis 4, **dadurch gekennzeichnet, dass** es sich bei dem Polyol um 1,3-Dihydroxy-2,2-di(hydroxymethyl)propan (Pentaerythrit) handelt.

6. Pulverlackzusammensetzung nach Anspruch 1, **dadurch gekennzeichnet, dass**
A einer Gruppe der Formel (III) entspricht

7. Pulverlackzusammensetzung nach Anspruch 6, **dadurch gekennzeichnet, dass**
x 3 bis 6, vorzugsweise 3, bedeutet

8. Pulverlackzusammensetzung nach einem der Ansprüche 6 oder 7, **dadurch gekennzeichnet, dass**
E einen Rest darstellt, abgeleitet von einem aliphatischen Polyol mit 3 bis 20 Kohlenstoffatomen.

9. Pulverlackzusammensetzung nach Anspruch 8, **dadurch gekennzeichnet, dass** das Polyol ausgewähit ist aus Mannit, insbesondere D-Mannit, Sorbit, Insbesondere D-Sorbit, und Dulcit.

10. Pulverlackzusammensetzung nach Anspruch 1, **dadurch gekennzeichnet, dass**
A der Gruppe der Formel (IV) entspricht.

11. Pulverlackzusammensetzung nach einem der Ansprüche 1 bis 10, **dadurch gekennzeichnet, dass** sie weiterhin mindestens eine bei 25°C feste Epoxidverbindung der Formel (I) enthält, worin
A einer Gruppe der Formel (II) oder (III) entspricht und
x 2 bedeutet.

12. Pulverlackzusammensetzung nach Anspruch 11, **dadurch gekennzeichnet, dass** die Epoxidverbindungen der Formel (I), worin x mindestens 3 ist, und die Epoxidverbindungen der Formel (I), worin x gleich 2 ist, in einem molaren Verhältnis bis zu maximal 1:2, vorzugsweise bis zu maximal 1:1 vorliegen.

13. Pulverlackzusammensetzung nach einem der Ansprüche 1 bis 12, die weitgehend frei von Glycidylverbindungen, insbesondere von TGIC und Glycidylestem, isf.

## Claims

1. A powder coating composition comprising a binder selected from carboxyl-group-containing polyesters, carboxyl-group-containing poly(meth)acrylates and mixtures of the said substances, and one or more epoxy compounds, wherein the epoxy compounds comprise at least one compound of formula (I) that is solid at 25°C: 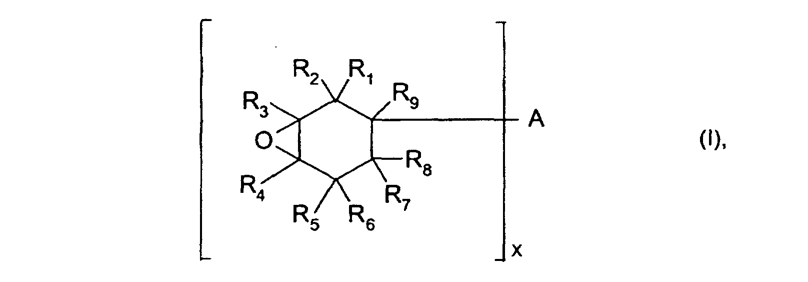 wherein
A corresponds to a group of formula (II), (III), (IV) or (VI): 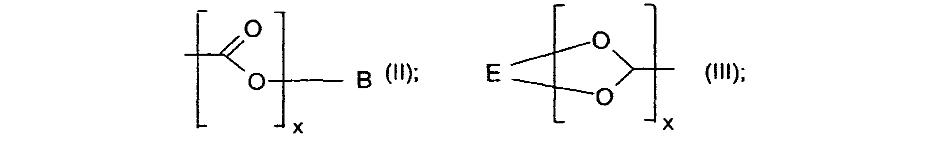 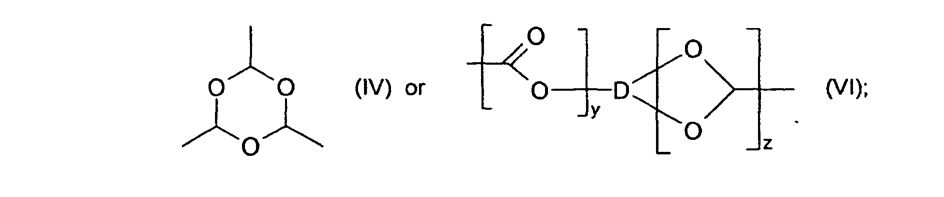
in which
B is an x-valent organic radical that is derived from a polyol having x or more hydroxyl groups by the removal of x hydroxyl groups;
E is a (2x)-valent organic radical that is derived from a polyol having (2x) or more hydroxyl groups by the removal of (2x) hydroxyl groups; and
D is a (y + 2z)-valent radical that is derived from a polyol having (y + 2z) or more hydroxyl groups by the removal of (y + 2z) hydroxyl groups;
R₁ and R₅ are each independently of the other hydrogen, halogen, C₁-C₄alkyl or C₁-C₄alkoxy or are together a methylene group; and
R₂, R₃, R₄, R₆, R₇, R₈ and R₉ are each independently of the others hydrogen, halogen, C₁-C₄alkyl or C₁-C₄alkoxy; and
x is an integer of at least 3;
y is an integer from 1 to (x - 1) and
z is (x - y).

2. A powder coating composition according to claim 1, wherein A corresponds to a group of formula (II).

3. A powder coating composition according to claim 2, wherein x is from 3 to 6, preferably 4.

4. A powder coating composition according to claim 3, wherein B is a radical that is derived from an aliphatic polyol having from 3 to 20 carbon atoms, a cycloaliphatic polyol having from 5 to 20 carbon atoms or a mixed aliphatic-cycloaliphatic polyol having from 7 to 20 carbon atoms.

5. A powder coating composition according to claim 1, wherein the polyol is 1,3-dihydroxy-2,2-di(hydroxymethyl)propane (pentaerythritol).

6. A powder coating composition according to claim 1, wherein A corresponds to a group of formula (III).

7. A powder coating composition according to claim 6, wherein x is from 3 to 6, preferably 3.

8. A powder coating composition according to either claim 6 or claim 7, wherein E is a radical that is derived from an aliphatic polyol having from 3 to 20 carbon atoms.

9. A powder coating composition according to claim 8, wherein the polyol is selected from mannitol, especially D-mannitol, sorbitol, especially D-sorbitol, and dulcitol.

10. A powder coating composition according to claim 1, wherein A corresponds to a group of formula (IV).

11. A powder coating composition according to claim 1, that furthermore comprises at least one epoxy compound of formula (I) that is solid at 25°C in which
A corresponds to a group of formula (II) or (III) and
x is 2.

12. A powder coating composition according to claim 11, wherein the epoxy compounds of formula (I) wherein x is at least 3 and the epoxy compounds of formula (I) wherein x is 2 are present in a molar ratio of up to a maximum of 1:2 preferably of up to a maximum of 1:1.

13. A powder coating composition according to claim 1, which is substantially free of glycidyl compounds, especially TGIC and glycidyl esters.

## Revendications

1. Composition de vernis en poudre contenant un liant, qui est pris parmi un polyester présentant des groupes carboxyle, un poly(méth)acrylate présentant des groupes carboxyle et des mélanges des substances précitées, et un ou plusieurs composés époxydes, **caractérisée en ce que** les composés époxydes comprennent au moins un composé solide à 25°C de formule (I) : 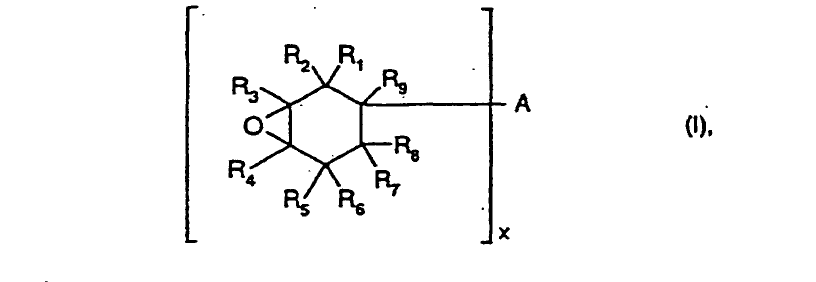 où
A représente un groupe de formule (II), (III), (IV) ou (VI) : 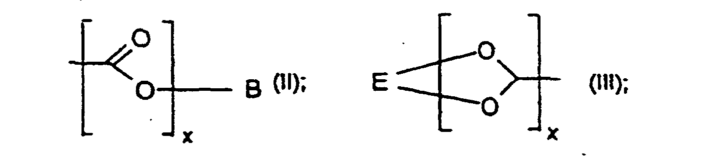 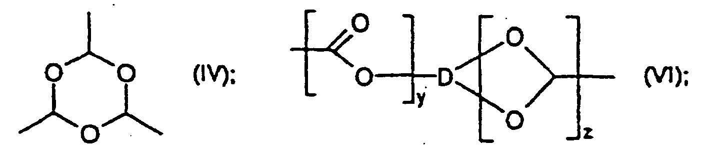
où
B représente un reste organique x-valent dérivant d'un polyol avec x ou plus de groupes hydroxyle par élimination de x groupes hydroxyle ;
E représente un reste organique (2x)-valent dérivant d'un polyol avec (2x) ou plus de groupes hydroxyle par élimination de (2x) groupes hydroxyle ;
et
D représente un reste (y + 2z), dérivant d'un polyol avec (y + 2z) ou plus de groupes hydroxyle par élimination de (y + 2z) de groupes hydroxyle ;
R₁ et R₅ représentent, indépendamment l'un de l'autre, un atome d'hydrogène, un atome d'halogène, un groupe alkyle en C₁-C₄ ou alkoxy en C₁-C₄ ou conjointement un groupe méthylène ; et
R₂, R₃, R₄, R₆, R₇, R₈ et R₉ représentent indépendamment les uns des autres, un atome d'hydrogène, un atome d'halogène, un groupe alkyle en C₁-C₄ ou alkoxy en C₁-C₄ ; et
x est un entier d'au moins 3 ;
y est un entier de 1 à (x-1) et
z représente (x - y).

2. Composition de vernis en poudre selon la revendication 1, **caractérisée en ce que**
A représente un groupe de formule (II).

3. Composition de vernis en poudre selon la revendication 2, **caractérisée en ce que**
x va de 3 à 6, de préférence vaut 4.

4. Composition de vernis en poudre selon la revendication 3, **caractérisée en ce que**
B représente un reste, qui dérive d'un polyol aliphatique présentant 3 à 20 atomes de carbone, d'un polyol cycloaliphatique présentant 5 à 20 atomes de carbone ou d'un polyol mixte aliphatique-cycloaliphatique présentant 7 à 20 atomes de carbone.

5. Composition de vernis en poudre selon l'une des revendications 1 à 4, **caractérisée en ce qu'**il s'agit pour le polyol du 1,3-dihydroxy-2,2-di(hydroxyméthyl)propane (pentaérythrite).

6. Composition de vernis en poudre selon la revendication 1, **caractérisée en ce que**
A représente un groupe de formule (III).

7. Composition de vernis en poudre selon la revendication 6, **caractérisée en ce que**
x va de 3 à 6, de préférence vaut 3.

8. Composition de vernis en poudre selon l'une des revendications 6 ou 7, **caractérisée en ce que**
E représente un reste dérivant d'un polyol aliphatique présentant 3 à 20 atomes de carbone.

9. Composition de vernis en poudre selon la revendication 8, **caractérisée en ce que** le polyol est pris parmi le mannitol, notamment le D-mannitol, le sorbitol, notamment le D-sorbitol et le dulcitol.

10. Composition de vernis en poudre selon la revendication 1 **caractérisée en ce que**
A représente un groupe de formule (IV).

11. Composition de vernis en poudre selon l'une des revendications 1 à 10, **caractérisée en ce qu'**elle renferme en plus au moins un composé époxyde solide à 25°C de formule (I), où
A représente un groupe de formule (II) ou (III) et
x vaut 2.

12. Composition de vernis en poudre selon la revendication 11, **caractérisée en ce que** le composé époxyde de formule (I), où x vaut au moins 3, et les composés époxydes de formule (I) où x vaut 2, sont présents dans un rapport molaire allant au maximum jusqu'à 1:2, de préférence au maximum jusqu'à 1:1.

13. Composition de vernis en poudre selon l'une des revendications 1 à 12, qui est essentiellement exempte de composés glycidyliques, en particulier de TGIC et d'esters glycidyliques.
